(19) Europäisches Patentamt
European Patent Office
Office européen des brevets

(11) Veröffentlichungsnummer: 0 137 968
B1

(12) EUROPÄISCHE PATENTSCHRIFT

(45) Veröffentlichungstag der Patentschrift: 09.03.88

(51) Int. Cl.⁴: C 07 C 69/08, C 07 C 69/16, C 07 C 69/28, C 07 C 67/04

(21) Anmeldenummer: 84109852.8

(22) Anmeldetag: 18.08.84

# (54) Verfahren zur Herstellung von 1-Arylpropandiol-1,3-diacylaten.

(30) Priorität: 12.10.83 DE 3337102

(43) Veröffentlichungstag der Anmeldung: 24.04.85 Patentblatt 85/17

(45) Bekanntmachung des Hinweises auf die Patenterteilung: 09.03.88 Patentblatt 88/10

(84) Benannte Vertragsstaaten: BE CH DE FR GB IT LI NL

(56) Entgegenhaltungen:
DE - C - 965 696

JOURNAL OF THE AMERICAN CHEMICAL SOCIETY, Band 76, Nr. 10, 26. Mai 1954, Seiten 2685-2688, Columbus, Ohio, US; D. COFFMAN et al.: "The ionic telomerization of styrene"
CHEMICAL ABSTRACTS, Band 95, Nr. 11, 14. September 1981, Seite 610, Spalte 1, Abstract Nr. 97345p, Columbus, Ohio, US
CHEMICAL ABSTRACTS, Band 97, Nr. 21, 22. November 1982, Seite 785, Spalte 1, Abstract Nr. 181990u, Columbus, Ohio, US

(73) Patentinhaber: HÜLS AKTIENGESELLSCHAFT, - RSP Patente / PB 15 - Postfach 13 20, D-4370 Marl 1 (DE)

(72) Erfinder: Kleine Homann, Walter, Dr., Buchenallee 14, D-4408 Dülmen (DE)
Erfinder: Zöllner, Manfred, Landwehr 26, D-4350 Recklinghausen (DE)

EP 0 137 968 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

1-Arylpropandiol-1,3-diacylate, beispielsweise das 1-Phenylpropandiol-1,3-diacetat, erhält man nach der Prins-Reaktion durch Anlagerung von Formaldehyd an Styrol in essigsaurer Lösung in Gegenwart von Schwefelsäure (J. Amer. Chem. Soc. 70, 873 (1948).

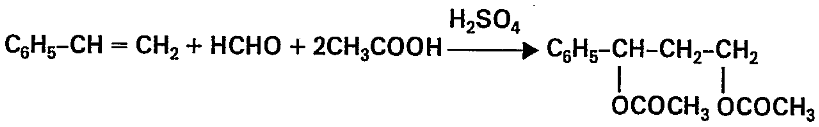


Als Nebenprodukt entsteht jedoch in grösseren Mengen 4-Phenyl-dioxan-1,3 (C.A. 54, 19688 h).

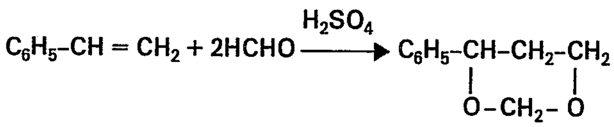


Zudem kann nach eigenen Versuchen als weiterer Zwangsanfall Methylendiacetat entstehen. Aus dieser Mischung ist das 1-Phenylpropandiol-1,3-diacetat nur schwierig zu gewinnen.

Nach einem weiteren Verfahren erhält man 1-Phenylpropandiol-1,3-diacetat durch Umsetzung von 4-Phenyl-dioxan-1,3 mit Acetanhydrid in Gegenwart von Eisen (III)- oder Zinkchlorid bzw. deren Gemische, gegebenenfalls unter Zusatz weiterer saurer Komponenten, wie Chlorwasserstoff, Schwefel- oder Phosphorsäure (CS-PS 185 517). Für dieses Verfahren ist jedoch als Vorstufe die Herstellung von 4-Phenyl-dioxan-1,3 erforderlich.

Hieraus ergab sich die Aufgabe, ein Verfahren zu finden, das die Herstellung von 1-Arylpropandiol-1,3-diacylaten in einfacher und wirtschaftlicher Weise ermöglicht.

Diese Aufgabe wird erfindungsgemäss entsprechend den Angaben der Patentansprüche gelöst.

Überraschenderweise erhält man 1-Arylpropandiol-1,3-diacylate in hoher Ausbeute, wenn man Arylolefine mit Carbonsäuren und/oder deren Anhydriden und Formaldehyd bezogen auf Arylolefin, in Molverhältnissen von 1:2 bis 10:0,5 bis 2, vorzugsweise 1:3 bis 6:0,7 bis 1 in Gegenwart eines sauren Katalysators umsetzt und dem Reaktionsgemisch 4-Aryl-dioxan-1,3 zusetzt. Der Zusatz von 4-Aryl-dioxanen-1,3 verhindert überraschenderweise den Zwangsanfall von 4-Aryl-dioxanen-1,3. Die 4-Aryl-dioxane-1,3 setzt man in Molverhältnissen von 0,1 bis 1 Mol, insbesondere 0,2 bis 0,4 Mol 4-Aryl-dioxan-1,3/Mol/Arylolefin ein. Innerhalb der erfindungsgemässen Molverhältnisse entsteht zudem auch kein Methylendiacylat.

Die Umsetzung erfolgt bei Temperaturen von 10 bis 50°. Bevorzugt arbeitet man bei Temperaturen von 20 bis 40°C, insbesondere 25 bis 30°C. Im allgemeinen arbeitet man bei Normaldruck, man kann aber auch bei Über- oder Unterdruck arbeiten.

Als Arylolefin sind beispielsweise Styrol, substituierte Styrole, wie Methyl- und Ethylstyrole geeignet. Bevorzugt setzt man Styrol und Methylstyrol ein.

Geeignete Carbonsäuren bzw. deren Anhydride sind beispielsweise Ameisensäure, Essigsäure, Propionsäure, Buttersäure, Isobuttersäure, Valeriansäure, Capronsäure, Benzoesäure, alkylierte Benzoesäuren wie tert.-Butylbenzoesäure.

Bevorzugt setzt man Essigsäure, Propionsäure oder Isobuttersäure ein, insbesondere Mischungen der Carbonsäuren mit deren Anhydriden, wobei man bevorzugt Molverhältnisse von Formaldehyd zu Carbonsäuren und deren Anhydriden von 1:2 bis 10, insbesondere 1:3 bis 5, wählt.

Als saure Katalysatoren sind beispielsweise Schwefelsäure, p-Toluolsulfonsäure, Friedel-Crafts-Katalysatoren und Ionenaustauscher geeignet. Bevorzugt setzt man konzentrierte Schwefelsäure oder Ionenaustauscher ein.

Als Formaldehyd kann man ausser dem monomeren Formaldehyd auch Paraformaldehyd einsetzen. Geeignete 4-Aryl-dioxane-1,3 sind beispielsweise 4-Phenyldioxan-1,3 substituierte 4-Phenyldioxane-1,3 wie 4-Tolyl-dioxane-1,3 und 4-[Ethyl-phenyl]-dioxane-1,3.

Bevorzugt setzt man 4-Phenyldioxan-1,3 ein.

Die Reaktion ist im allgemeinen nach 2 bis 12 Stunden beendet. Durch eine Nachreaktion, im allgemeinen in einer Zeit von 8 bis 48 Stunden, kann man den Umsatz erhöhen.

Nach der Umsetzung neutralisiert man gegebenenfalls die erhaltene Reaktionslösung, beispielsweise mit einer Alkalihydroxidlösung, z.B. einer 10 bis 50%igen Lösung von NaOH. Die weitere Aufarbeitung erfolgt im allgemeinen durch Rektifikation.

Nach dem erfindungsgemässen Verfahren erhält man die 1-Aryl-propandiol-1,3-diacylate bei hohem Umsatz in hoher Selektivität. In geringer Menge können als Nebenprodukte 1-Aryl-propandiol-mono-acylate und Zimtalkoholacylate entstehen. Der Monoacylanteil kann durch Zugabe des entsprechenden Anhydrids vor der Destillation zum Diacylat umgesetzt werden.

Die 1-Arylpropandiol-1,3-diacylate, vorzugsweise die 1-Phenylpropandiol-1,3-diacylate, insbesondere die -diacetate, -dipropionate und -diisobutyrate, sind als Vorstufe für die Synthese von Zimtalkoholestern und damit für die Herstellung von Riechstoffen von grossem Interesse.

Vergleichsbeispiel A

Zu einem Gemisch bestehend auf 300 g (5 Mol) Essigsäure, 150 g (1,5 Mol) Essigsäureanhydrid, 75 g (2,5 Mol) Formaldehyd und 25 g (0,25 Mol) Schwefelsäure werden bei ca. 30°C 260 g (2,5 Mol) Styrol dosiert. Nach beendeter Reaktion wird die Reaktionslösung auf einen pH-Wert von ca. 2 gestellt. Durch Destillation des Rohaustrages erhält man neben 82 g (0,5 Mol) 4-Phenyl-dioxan-1,3, 248 g (1,05 Mol) 1-Phenylpropandiol-1,3-diacetat. An Rückstyrol werden 31,5 g (0,3 Mol) und an Essigsäure 270 g (4,5 Mol) gewonnen. Die Reinheit des Diacetats beträgt > 98%.

Beispiel 1

Zu einem Gemisch bestehend aus 1200 g (20 Mol) Essigsäure, 610 g (6 Mol) Anhydrid, 210 g (7 Mol) Formaldehyd, 98 g (1 Mol) Schwefelsäure und 328 g (2 Mol) 4-Phenyldioxan-1,3 werden bei ca. 30°C 1040 g (10 Mol) Styrol dosiert. Nach beendeter Reaktion wird die Reaktionslösung auf einen ph-Wert von ca. 2 gestellt. Durch Destillation des Rohaustrages erhält man neben 160 g (1,95 Mol) 4-Phenyldioxan-1,3 1404 g (5,95 Mol) 1-Phenylpropandiol-1,3-diacetat. An Rückstyrol werden 166 g (1,6 Mol) und an Essigsäure 1020 g (17 Mol) gewonnen.

Die Reinheit des Diacetats beträgt > 99%.

Beispiel 2

Zu einem Gemisch bestehend aus 1485 g (20 Mol) Propionsäure, 780 g (6 Mol) Propionsäureanhydrid, 328 g (2 Mol) 4-Phenyldioxan-1,3, 210 g Formaldehyd und 98 g (1 Mol) Schwefelsäure werden bei ca. 30°C 1040 g (10 Mol) Styrol dosiert. Nach beendeter Reaktion wird die Reaktionslösung auf einen pH-Wert von ca. 2 gestellt. Durch Destillation des Rohaustrages erhält man neben 352 g (2,2 Mol) 4-Phenyldioxan-1,3 1530 g (5,8 Mol) 1-Phenyl-propandiol-1,3-dipropionat. An Rückstyrol werden 192 g (1,8 Mol) und an Propionsäure 1400 g (18,9 Mol) gewonnen.

Die Reinheit des Dipropionats beträgt > 98%.

Beispiel 3

Zu einem Gemisch bestehend aus 1760 g (20 Mol) Isobuttersäure, 948 g (6 Mol) Isobuttersäureanhydrid, 328 g (2 Mol) 4-Phenyldioxan-1,3 210 g (7 Mol) Formaldehyd und 98 g (1 Mol) Schwefelsäure werden bei ca. 30°C 1 040 g (10 Mol) Styrol dosiert. Nach beendeter Reaktion wird die Reaktionslösung auf einen pH-Wert von ca. 2 gestellt. Durch Destillation des Rohaustrages erhält man neben 346 g (2,1 Mol) 4-Phenyldioxan-1,3 1 110 g (3,8 Mol) 1-Phenylpropandiol-1,3-diisobutyrat. An Rückstyrol werden 312 g (3 Mol) und an Isobuttersäure 1 800 g (20,4 Mol) gewonnen.

Die Reinheit des Diisobutyrats beträgt > 98%.

Beispiel 4

Zu einem Gemisch bestehend aus 460 g (10 Mol) Ameisensäure, 164 g (1 Mol) 4-Phenyldioxan-1,3, 105 g (3,5 Mol) Formaldehyd und 49 g (0,5 Mol) Schwefelsäure werden bei ca. 30°C 520 g (5 Mol) Styrol dosiert. Nach beendeter Reaktion wird die Reaktionslösung auf einen pH-Wert von ca. 1,5 gestellt. Durch Destillation des Rohaustrages erhält man neben 225 g (1,4 Mol) 4-Phenyldioxan-1,3 391 g (1,9 Mol) 1-Phenylpropandiol-1,3-diformiat.

An Rückstyrol werden 205 g (2,0 Mol) und an Ameisensäure 82 g (1,8 Mol) gewonnen.

Die Reinheit des Diformiats beträgt > 97%

Vergleichsbeispiel B

Zu einem Gemisch bestehend aus 240 g (4 Mol) Essigsäure, 510 g (5 Mol) Essigsäureanhydrid, 150 g (5 Mol) Formaldehyd und 49 g (0,5 Mol) Schwefelsäure werden bei ca. 30°C 520 g (5 Mol) Styrol dosiert. Nach beendeter Reaktion wird die Reaktionslösung auf einen pH-Wert von ca. 2 gestellt. Durch Destillation des Rohaustrages erhält man neben 77 g (0,5 Mol) 4-Phenyl-dioxan-1,3 430 g (1,8 Mol) 1-Phenylpropandiol-1,3-diacetat nach 322 g (2,4 Mol) Methylendiacetat. An Rückstyrol werden 75 g (0,7 Mol) und an Essigsäure 276 g (4,6 Mol) gewonnen.

Beispiel 5

Zu einem Gemisch bestehend aus 600 g (10 Mol) Essigsäure, 306 g (3 Mol) Essigsäureanhydrid, 105 g (3,5 Mol) Formaldehyd, 178 g (1 Mol) 4-Tolyldioxan-1,3 und 49 g (0,5 Mol) Schwefelsäure werden bei ca. 30°C 590 g (5 Mol) Methylstyrol dosiert.

Nach beendeter Reaktion wird die Reaktionslösung auf einen pH-Wert von ca. 2 gestellt. Durch Destillation des Rohaustrages erhält man neben 169 g (0,95 Mol) 4-Tolyldioxan-1,3 800 g (3,2 Mol) 1-Tolylpropandiol-1,3-diacetat. An Rückmethylstyrol werden 24 g (0,2 Mol) und an Essigsäure 510 g (8,5 Mol) gewonnen.

**Patentansprüche**

1. Verfahren zur Herstellung von 1-Arylpropandiol-1,3-diacylaten durch Umsetzung von Arylolefinen mit Carbonsäuren und/oder deren Anhydriden und mit Formaldehyd in Gegenwart von Säuren, dadurch gekennzeichnet, dass man Arylolefine, Carbonsäuren und/oder deren Anhydride sowie Formaldehyd in Molverhältnissen von 1 : 2 bis 10 : 0,5 bis 2 einsetzt und dem Reaktionsgemisch 0,1 bis 1 Mol 4-Aryl-dioxan-1,3/Mol Arylolefin zusetzt und bei Temperaturen von 10 bis 50°C umsetzt.

2. Verfahren nach Anspruch 1, dadurch gekennzeichnet, dass man Arylolefine, Carbonsäuren und/oder deren Anhydride sowie Formaldehyd in Molverhältnissen von 1 : 3 bis 6 : 0,7 bis 1 einsetzt.

3. Verfahren nach den Ansprüchen 1 und 2, dadurch gekennzeichnet, dass man Formaldehyd zu den Carbonsäuren und deren Anhydriden in Molverhältnissen von 1 : 2 bis 10 einsetzt.

4. Verfahren nach den Ansprüchen 1 bis 3, dadurch gekennzeichnet, dass man als Arylolefine Styrol oder Methylstyrol einsetzt.

5. Verfahren nach den Ansprüchen 1 bis 4, dadurch gekennzeichnet, dass man als Carbonsäuren Ameisensäure, Essigsäure, Propionsäure, Buttersäure, iso-Buttersäure bzw. deren Anhydride einsetzt.

6. Verfahren nach den Ansprüchen 1 bis 5, dadurch gekennzeichnet, dass man als 4-Aryl-dioxan-1,3 4-Phenyl-dioxan-1,3 oder 4-Tolyl-dioxan-1,3 einsetzt.

**Claims**

1. Process for preparation of 1-arylpropanediol-1,3-diacylates by reaction of arylolefines with carboxylic acids and/or their anhydrides and with formaldehyde in the presence of acids, characterized in that, the arylolefine, carboxylic acids and/or their anhydrides and formaldehyde are used in molar ratios of 1:2 to 10:0.5 to 2, and 0.1 to 1 Mol 4-aryl-1,3-dioxane/Mol arylolefine is added to the reaction mixture and reacted at temperatures of 10 to 50°C.

2. Process according to Claim 1, characterized in that, the arylolefine, carboxylic acids and/or their anhydrides and formaldehyde are used in molar ratios of 1:3 to 6:0.7 to 1.

3. Process according to Claims 1 and 2, characterized in that, formaldehyde is added to the carboxylic acids and their anhydrides in molar ratios of 1:2 to 10.

4. Process according to Claims 1 to 3, characterized in that, as arylolefines, styrene or methylstyrene are used.

5. Process according to Claims 1 to 4, characterized in that, as carboxylic acids, formic acid, acetic acid, propionic acid, butyric acid, isobutyric acid or their anhydrides are used.

6. Process according to Claim 1 to 4, characterized in that, as 4-aryl-1,3-dioxane, 4-phenyl-1,3-dioxane or 4-tolyl-1,3-dioxane is used.

**Revendications**

1. Procédé pour la préparation de diacylates de 1-arylpropanediol-1,3 par réaction d'aryloléfines avec des acides carboyliques et/ou leurs anhydrides et avec le formaldéhyde en présence d'acides, caractérisé en ce que l'on met en réaction des aryloléfines, des acides carboxyliques et/ou leurs anhydrides ainsi que du formaldéhyde en proportions molaires de 1:2 à 10:0,5 à 2, et on ajoute au mélange réactionnel 0,1 à 1 mole de 4-aryldioxanne-1,3 par mole d'aryloléfine, et on le fait réagir à des températures de 10 à 50°C.

2. Procédé selon la revendication 1, caractérisé en ce que l'on met en réaction des aryloléfines, des acides carboxyliques et/ou leurs anhydrides ainsi que du formaldéhyde en proportions molaires de 1:3 à 6:0,7 à 1.

3. Procédé selon les revendications 1 et 2, caractérisé en ce que l'on utilise des proportions molaires du formaldéhyde aux acides carboxyliques et leurs anhydrides allant de 1:2 à 10.

4. Procédé selon l'une quelconque des revendications 1 à 3, caractérisé en ce que l'on utilise en tant qu'aryloléfines le styrène ou le méthylstyrène.

5. Procédé selon l'une quelconque des revendications 1 à 4, caractérisé en ce que l'on utilise en tant qu'acides carboxyliques l'acide formique, l'acide acétique, l'acide propionique, l'acide butyrique, l'acide isobutyrique ou leurs anhydrides.

6. Procédé selon l'une quelconque des revendications 1 à 5, caractérisé en ce que l'on utilise en tant que 4-aryl-dioxanne-1,3, le 4-phényldioxanne-1,3 ou le 4-tolyldioxanne-1,3.